# EUROPEAN PATENT SPECIFICATION

(11) **EP 4 554 557 B1**
(45) Date of publication and mention of the grant of the patent: **11.03.2026**
(21) Application number: 23734323.1
(22) Date of filing: 29.06.2023
(51) Int. Cl.: A61K 9/00, A61L 31/04, A61L 31/16, A61K 47/10, A61K 47/34

(54) **DEVICE FOR SUSTAINED CONTROLLED RELEASE OF A THERAPEUTICALLY ACTIVE SUBSTANCE AND USES THEREOF**
VORRICHTUNG ZUR VERZÖGERTEN KONTROLLIERTEN FREISETZUNG EINES THERAPEUTISCHEN WIRKSTOFFS UND VERWENDUNGEN DAVON
DISPOSITIF DE LIBÉRATION CONTRÔLÉE PROLONGÉE D'UN PRINCIPE ACTIF THÉRAPEUTIQUE ET SES UTILISATIONS

(30) Priority: 11.07.2022 EP 22184123
(43) Date of publication of application: 21.05.2025
(73) Proprietor: Hylomorph AG, 8005 Zürich (CH)
(72) Inventor: FERRARI, Aldo, 8057 Zürich (CH); BOTTAN, Simone, 8048 Zürich (CH); ROBOTTI, Francesco, 8057 Zürich (CH); STEFOPOULOS, Georgios, 8041 Zürich (CH)
(74) Representative: Bremi, Tobias Hans
(86) International application number: PCT/EP2023/067780
(87) International publication number: WO 2024/012883

(56) References cited:
- KR-B1- 100 376 083
- US-A1- 2015 196 497
- TERRY W J STEELE ET AL: "The effect of polyethylene glycol structure on paclitaxel drug release and mechanical properties of PLGA thin films", ACTA BIOMATERIALIA, ELSEVIER, AMSTERDAM, NL, vol. 7, no. 5, 2 February 2011 (2011-02-02), pages 1973 - 1983, XP028188269, ISSN: 1742-7061, [retrieved on 20110205], DOI: 10.1016/J.ACTBIO.2011.02.002

## Description

### TECHNICAL FIELD

The present invention relates to devices, in particular films or layers for sustained controlled release of a therapeutically active substance as well as to methods for making such devices and uses of such devices.

### PRIOR ART

Non-degradable implantable devices, implanted to stay in place, need to be structured to avoid a negative foreign body response of the patient. This applies to fully embedded implants such as breast implants or pacemakers, but also implants that require long-term trans dermal communication. Certain types of implantable medical devices require long-term trans dermal communication between the internal and external portions of the device. Examples of such devices include left ventricular assist devices, tissue expanders, arteriovenous shunts, and gastric lap bands. The transdermal portion of the device can be in the form of a catheter, a gas insufflation tube, or one or several electrical wires. These types of transdermal medical devices are susceptible to infection.

In order to increase the compatibility between the surrounding tissue and the actual implantable medical device it is possible to provide for a pouch into which the implantable medical device is put before insertion into the body, providing an optimum interface between the surrounding tissue and the implant avoiding complications and assisting healing in of the device.

A problem with implantable medical devices, in particular those with long-term transdermal communication, is that there is a high infection rate after implantation. Typically, this is treated by oral or intravenous administration of antibiotics, alone or combined with local flushing with antibiotic solution, however in particular in case of implants located at physiologically delicate places in the body which at the same time are not quickly accessible for orally administered antibiotics, this may not be sufficient.

There remains a need to reduce the incidence of infection associated with implantable devices.

Wound dressings are designed to support the wounded region, protect it from infection, and, in certain cases, actively promote wound healing by creating a favorable environment for cell growth.

The response to wounding, defined as a breakage of bodily tissue, involves an inflammation phase, a migratory phase and a remodeling phase. The inflammation phase is the acute response to a wound and its purpose is to quickly seal the wound and produce chemical factors that employ cells to migrate into the wound and start the wound healing process. During the migratory phase, cells rapidly migrate into the wound and start laying down provisional extracellular matrix that will be the base of the healed tissue. During the remodeling stage, the newly created tissue slowly matures into its permanent form.

Standard wound dressings facilitate wound healing by: 1. mechanically holding the wound edges together to allow easier cell migration; 2. mechanically sealing the wound to prevent contamination by pathogens; 3. in some advanced dressings providing an environment that actively promotes faster wound healing, usually by exposing the wounded tissue to a hydrated gel. Improved materials for these applications would be desirable.

In plastic surgery commercial silicone implants (e.g. breasts, calf, buttock, chest, biceps), but also the above-mentioned implantable medical devices often fail due to foreign body reaction and scar-tissue encapsulation. Also for such applications improved materials/coatings would be desirable.

In cosmetics disposable flat and unstructured cellulose masks are available (e.g. face, hands and feet) as masks that enhance skin hydration, as well as the absorption of metabolic waste products and the release of nutrients or other compounds to the skins.

WO2015040106A1 proposes a method for the self-assembled production of a topographically surface structured cellulose element which can be used as a material for such a pouch, wherein in a first step a mold with on one side a first surface which is in a complementary manner topographically surface structured and which is permeable to oxygen is provided, wherein a liquid growth medium containing cellulose producing bacteria is provided, and wherein the mold is placed to form a liquid/air interface of the liquid growth medium such that the side of the mold with the first surface is in direct contact with the liquid growth medium, and with an opposite side is facing air or a specifically provided oxygen containing gas surrounding, allowing for said bacteria to produce and deposit cellulose on said first surface and developing on the interface therewith a topographically surface structured surface complementary thereto, until a contiguous cellulose layer with a thickness of the element of at least 0.3mm is formed; and wherein in a second step the element is removed from said mold. Furthermore, the invention relates to elements made using such a method and uses of such elements for various applications. The corresponding pouch provides for optimum compatibility to the surrounding tissue directly after implantation but also over longer time spans when the implant is to remain in the body.

US-A-2019009063 discloses an inflatable balloon which is enclosed by an expandable cover which becomes increasingly porous/permeable during expansion. The balloon is coated or enclosed with a matrix which contains a pharmaceutically active agent. During expansion of the balloon, the pharmaceutically active agent is released or extruded through the expandable cover into a body cavity such as an artery or vein.

US-A-2007141106 provides a medical system for the administration of a pharmaceutical agent in vivo to a patient. The medical system includes a medical implant positionable in a body of a patient. A pharmaceutical agent is disposed on the medical implant and at least partially coated with a reactive coating. The reactive coating acts to control the release of the pharmaceutical agent. An energy unit is provided for transmitting an energy signal to the reactive coating, wherein the reactive coating reacts to the energy signal to increase the release rate of the pharmaceutical agent.

WO-A-2008136856 describes biodegradable and resorbable polymer pouches for use with cardiac rhythm management devices (CRMs) and other implantable medical devices (IMDs), i.e., a pouch, covering, or other receptacle capable of encasing, surrounding and/or holding the CRM or other IMD for the purpose of securing it in position, inhibiting or reducing bacterial growth, providing pain relief and/or inhibiting scarring or fibrosis on or around the CRM or other IMD. Optionally, the biodegradable and resorbable pouches of the invention include one or more drugs in the polymer matrix to provide prophylactic effects and alleviate side effects or complications associated with the surgery or implantation of the CRM or other IMD.

US-A-2020197712 describes nonwoven resorbable pouches that at least partially enclose implantable medical devices and improved methods for producing the implantable medical device pouches. The nonwoven pouches may comprise one or more drugs. Implantable medical devices that are placed in the pouches prior to implantation are prevented from migrating from the site of implantation by tissue ingrowth into the pouch. Antibiotics may be incorporated into the pouches to prevent post-operative infections. The pouches may be formed in fewer steps than conventional pouches, and without polymer coatings. Nonwoven pouches can be formed in one step by dry spinning instead of using multiple processing steps. In embodiments, the nonwoven pouches are smoother on the inside than the outside to tightly fit the implantable medical devices internally while encouraging external tissue ingrowth. In embodiments, the nonwoven pouches eliminate the use of knitted or woven multifilament fibers that can trap bacteria and result in post-operative infection.

Steele et al (Acta Biomater. 2011 May;7(5):1973-1983, doi: 10.1016/j.actbio.2011.02.002) report of thin films of poly(lactic acid-co-glycolic acid) (PLGA) incorporating paclitaxel which typically have slow release rates of paclitaxel of the order of 1 µg day(-1) cm(-2). For implementation as medical devices a range of zero order release rates (i.e. 1-15 µg day(-1) cm(-2)) is desirable for different tissues and pathologies. Eight and 35 kDa molecular weight polyethylene glycol (PEG) was incorporated at 15%, 25% and 50% weight ratios into PLGA containing 10 wt.% paclitaxel. The mechanical properties were assessed for potential use as medical implants and the rates of release of paclitaxel were quantified as per cent release and the more clinically useful rate of release in µg day(-1) cm(-2). Paclitaxel quantitation was correlated with the release of PEG from PLGA, to further understand its role in paclitaxel/PLGA release modulation. PEG release was found to correlate with paclitaxel release and the level of crystallinity of the PEG in the PLGA film, as measured by Raman spectrometry. This supports the concept of using a phase separating, partitioning compound to increase the release rates of hydrophobic drugs such as paclitaxel from PLGA films, where paclitaxel is normally homogeneously distributed/dissolved. Two formulations are promising for medical device thin films, when optimized for tensile strength, elongation, and drug release. For slow rates of paclitaxel release an average of 3.8 µg day(-1) cm(-2) using 15% 35k PEG for >30 days was achieved, while a high rate of drug release of 12 µg day(-1) cm(-2) was maintained using 25% 8 kDa PEG for up to 12 days.

KR-B-100376083 relates to a biocompatible and biodegradable polymer matrix for sustained release implant containing hydrophilic drug or its salt is provided, therefore the macromolecule matrix has improved drug-releasing rate and an initial burst effect. The biocompatible and biodegradable polymer matrix for sustained release implant containing hydrophilic drug or its salt is produced by the steps of: a) dissolving a first polymer selected from poly(d,1-lactide-co-glycolide(PLGA), polyglycolide(PGA) and polylactide(PLA), a second polymer of poly ethylene glycol(PEG) and a surfactant in an organic solvent; b) dissolving hydrophilic drug or its salt in water; adding the solution produced in the step (b) into the solution produced in the step a) and stirring them to produce water in oil(W/O) emulsion; and c) removing water and organic solvent from the W/O emulsion.

US-A-2015196497 describes a biocompatible polymeric controlled release matrix barrier structure for delivery of one or more bioactive agents from an implantable medical device. In an embodiment, a biocompatible polymeric controlled release matrix barrier structure is included. The biocompatible polymeric controlled release matrix can include a body structure formed of a compliant material comprising one or more compliant biocompatible polymers and one or more bioactive agents. The body structure can define a central aperture through which a subcutaneous element of an implantable medical device passes. Other embodiments are included herein.

### SUMMARY OF THE INVENTION

It is an object of the present invention to provide for improved and optimised devices for release, preferably sustained controlled release, of at least two therapeutically active substance after implantation of said device, either alone or in combination with other implantable elements. These implantable elements can be one or a combination of the following devices: artificial hips, artificial joints, artificial knees, bone fusion hardware, breast implants, cochlear implants, contraceptive Intra-Uterine Devices (IUDs), dental implants, ear tubes, implantable insulin pumps, implantable pulse generators, implantable sensors, joint fusion hardware, metal screws, muscle fusion hardware, pins, plates and rods, surgical meshes, orthopedic spacers, drivelines, catheters, so including pacemakers, neurostimulators, ventricular assist devices (VAD), or the like.

More specifically, the present invention according to a first aspect relates to a device as defined in claim 1.

Correspondingly, the present invention proposes a device for sustained controlled release, of therapeutically active substances after implantation of said device alone or in combination with further implantable elements. According to the invention, said device comprises or consists of at least one layer of material comprising at least two therapeutically active substances and providing for sustained controlled release of said substances under physiological conditions.

According to the invention, said layer comprises a matrix consisting of a mixture of
75-95 % by dry weight of a first component (A) in the form of a copolymer of DL-lactide and glycolide (poly(lactic-co-glycolic acid, PLGA) with a molar ratio of DL-lactide to glycolide in the range of 40:60-60:40 and with a weight average molecular weight MW_{w} in the range of 100,000-150,000 g/mol and
5-25% by dry weight of a second component (B) in the form of polyethylene glycol (PEG). The dry weights of component (A) and (B) are complementing to 100% of said matrix.

According to the invention, in this matrix the at least two therapeutically active substances are dissolved and/or suspended.

As will be shown in the following, this very particular matrix with these two components as defined provide for an optimum release and biodegradability profile for layers, in particular to be used in the corresponding layer to avoid post implantation inflammations when implanting non-degradable implants, but also for other purposes. Also, the proposed matrix allows to dissolve different types of therapeutically active substances, in particular it allows to at the same time dissolve and make available for controlled release therapeutically active substances which are water-soluble (hydrophilic) and water insoluble (hydrophobic). Correspondingly the proposed matrix is particularly suitable and adapted for a combined application with at least two different therapeutically active substances, for example Minocycline and Rifampicin. Furthermore, the proposed matrix provides for a surrounding stabilising the corresponding therapeutically active substances allowing for longer shelf life and preventing conversion/degradation of the API.

Herein we define hydrophilicity and hydrophobicity of a molecule, in particular of a therapeutically active substance, in reference to its solubility in water. Solubility is a chemical property of a molecule in a specific solvent which depends on the chemistry of the two and on the environmental conditions of temperature and pH. For the purpose of this document, we therefore define hydrophobicity as a low solubility of the molecule in water, specifically a solubility of less than 5 mg/ml, preferably of less than 3 mg/ml, and in particular of at most 2.5 mg/ml (but further preferably still larger than 0.1 mg/ml, or even larger than 0.5 mg/ml), and hydrophilicity as a high solubility of the molecule in water, specifically a solubility of more than 20 mg/ml, preferably of more than 40 mg/ml, and in particular of more than 45 mg/ml. This at room temperature (20 deg) or body temperature (37 deg) and a pH of 7. Solubility is measured in distilled and deionized water and is given in mg/mL.

According to the invention therefore in the matrix there are at least two therapeutically active substances, of which one is hydrophobic and one is hydrophilic. Preferably the hydrophobic active substance is selected as rifampin, and the hydrophilic active substance is selected as minocycline.

Preferably in this case the difference in the solubility in water is more than a factor of 10 (in milligrams/milliliter), more preferably more than a factor of 20, and most preferably more than a factor of 35 or in the range of 30 to 40.

The weight ratio of the respective therapeutically active substance to the PLGA is in each case in the range of 15-40%, preferably in the range of 20-35%. More preferably, the weight ratio of the hydrophilic therapeutically active substance, preferably minocycline, to PLGA is in the range of 15-30%, preferably in the range of 20-25%, and the weight ratio of the hydrophobic therapeutically active substance, preferably rifampin, to PLGA, is in the range of 25-40%, preferably in the range of 30-35%.

In fact, it was found that the proposed matrix turned out to be surprisingly suitable and adapted to take up at the time hydrophobic and a hydrophilic therapeutically active substances, and to controllably release both substances in a similar way as possible.

Minocycline is therefore hydrophilic as it can solve in water up to 50 mg/mL at neutral pH, i.e. pH of 7 at room temperature.

Rifampin is hydrophobic as it can solve in water only up to 2.5 mg/mL at neutral pH, i.e. pH of 7 at room temperature.

Therefore, there is a difference of 35 times in the water solubility of the two molecules under these conditions.

The key of the matrix is that, to load a sufficient amount of both types of molecules we need to exploit the different solubility of the two molecules in the PEG-PLGA structure.

Normally the proposed device in the form of a film or the like does not have a barrier function, in particular it cannot restore or substitute for example a skin barrier function. Furthermore, the proposed device is essentially inelastic and cannot be stretched for placement without tearing the device apart.

According to the invention, said first component (A) has a molar ratio of DL-lactide to glycolide in the range of 45:55-55:45, preferably in the range of 48:52-52:48, or in the range of about 50:50.

Further preferably, said first component (A) has a weight average molecular weight (MW_{w}) in the range of 110,000-140,000 g/mol, preferably in the range of 120,000-130,000 g/mol. Alternatively or additionally it is preferred if said first component (A) has a number average molecular weight (MWₙ) in the range of 50,000-100,000 g/mol, preferably in the range of 60,000-80,000 g/mol, most preferably in the range of 65,000-75,000 g/mol.

Alternatively or additionally preferably said first component (A) has an inherent viscosity midpoint in the range of 0.8-1.2 dl/g, preferably in the range of 0.9-1.1 dl/g (in each case measured in chloroform, 25 °C, c = 0.1 g/dl).

According to the invention, said second component (B) has a weight average molecular weight (MW_{w}) in the range of 3000-5500 g/mol, preferably in the range of 3200-4800 g/mol, most preferably in the range of 3700-4400 g/mol.

Typically and preferably, said second component (B) has a solidification point in the range of 45-68°C, preferably in the range of 50-65°C, most preferably in the range of 55-60°C.

As for the proportions, preferably said layer comprises a matrix consisting of a mixture of 75-92% by dry weight, preferably 80-90% by dry weight, most preferably 84-88% by dry weight of component (A) and
8-25% by dry weight, preferably 10-20% by dry weight, most preferably 12-16% by dry weight of component (B)
the dry weights of component (A) and (B) complementing to 100% of said matrix.

According to the invention at least two therapeutically active substances are selected from at least two antibiotics selected from the group of tetracyclines, penicillins, macrolides, ansamycines, wherein they are preferably selected from the group consisting of Tetracycline, Chlortetracycline, Oxytetracycline, Demeclocycline, Lymecycline, Meclocycline, Methacycline, Minocycline, Rolitetracycline, Doxycycline, Tigecycline, Eravacycline,Sarecycline, Omadacycline, Rifampicin, or a combination thereof, in particular a combination of Minocycline HCI and Rifampicin.

The concentration of the total of therapeutically active substances in the layer is in the range of of 25-40%, given as %w/w dry with respect to the total of the layer.

It is particularly preferred, if the layer as at least two therapeutically active substances comprise a combination of Minocycline, in particular in the form of Minocycline HCI, and Rifampicin in a total concentration of 20-40% by weight, preferably in the range of 25-35% by weight, in each case given as %w/w dry with respect to the total of the layer.

In this case preferably the concentration of Minocycline, in particular in the form of Minocycline HCI, is in the range of 10-20% by weight, more preferably in the range of 14-18%, and/or the concentration of Rifampicin is in the range of 15-25% by weight, preferably in the range of 17-22% by weight, in each case given as %w/w dry with respect to the total of the layer.

According to a particularly preferred embodiment, said layer comprises a matrix consisting of a mixture of
84-88% by dry weight of first component (A) with a weight average molecular weight in the range of 120,000-130,000 g/mol,
and 12-16% by dry weight of second component (B) with a weight average molecular weight MWw in the range of 3700-4400 g/mol,
the dry weights of component (A) and (B) complementing to 100% of said layer,
and wherein the layer as at least two therapeutically active substances comprise a combination of Minocycline, in particular in the form of Minocycline HCI, and Rifampicin in a total concentration of 25-35% by weight, wherein the concentration of Minocycline, in particular in the form of Minocycline HCl, is in the range of 14-18%, and the concentration of Rifampicin is in the range of 17-22% by weight, in each case given as %w/w dry with respect to the total of the layer.

Preferably for the envisaged applications, the device takes the form of at least one or only one self-supporting film, mesh, non-woven structure or combination thereof, preferably having a thickness in the range of 2.5-100 µm or 2.5 -50 µm, preferably in the range of 10-30 µm.

Further preferably, the device takes the form of a film or patch having a maximum extension in a lateral direction of 30 cm, preferably an extension in the range of 0.5-15 cm, more preferably in the range of 1-10 cm.

Such a film or patch preferably takes a round, oval or polygonal shape, preferably a rectangular or square shape, optionally with rounded edges.

The device may consist of one single or more than one of said layers, in combination with at least one additional, supporting layer (which supporting layer can be preferably biodegradable or non-degradable).

The device may take the form of a grid, nonwoven or woven or, preferably, of a contiguous layer. The device may also take the form of at least one strip, which is wrapped around a non-degradable implant in use. Furthermore the device may take the form of a pouch of the corresponding material or the form of a stretchable film like a clingfilm to completely wrap a non-degradable implant in use.

Said layer is preferably provided as at least two sheets which are put on opposing main faces of a non-degradable implantable device before insertion into said wet or humid cellulose pouch according to the use as specified further below.

Most preferably, the device consists of one single layer as defined above.

According to a further aspect of the present invention it relates to a method for making a device as defined above. According to this method, preferably in a first step said first component (A) is fully dissolved in an organic solvent, preferably selected from the group of acetonitrile, acetone, anisole, chloroform, dichloromethane, dimethylformamide, dimethylsulfoxide, ethyl acetate, dioxane, tetrahydrofuran, toluene, or a combination thereof, preferably under stirring at a temperature in the range of 15-30°C, preferably at room temperature (20-25°C), to firm a solution of said first component (A).

Further preferably, in a second step said second component (B) and the at least two therapeutically active substances are added to said solution of said first component (A). Again this preferably takes place under stirring at a temperature in the range of 15-30°C, preferably at room temperature, preferably in the same mixing device as for the first step. This is then followed by evaporation of the at least one solvent to form the device, preferably in a solution casting or solution coating process, preferably onto a release material.

According to a first preferred embodiment of the proposed method, after said first step and before carrying out said second step, a further organic solvent, different from the organic solvent used in the first step, is added to the solution of said first component (A), wherein preferably said further organic solvent is selected from the group of acetonitrile, chlorobenzene, chloroform, cumene, cyclohexane, 1,2-dichloroethene, dichloromethane,1,2-dimethoxyethane, N,N-dimethylacetamide, N,N-dimethylformamide, 1,4-dioxane, 2-ethoxyethanol, ethyleneglycol, formamide, hexane, methanol, 2-methoxyethanol, methylbutyl ketone, methylcyclohexane, methylisobutylketone, N-methylpyrrolidone, nitromethane, pyridine, sulfolane, tetrahydrofuran, tetralin, toluene, 1,1,2-trichloroethene, xylene, acetic acid, heptane, acetone, isobutyl acetate, anisole, isopropyl acetate, 1-butanol, methyl acetate, 2-butanol, 3-methyl-1-butanol, butyl acetate, methylethyl ketone, tert-butylmethyl ether, 2-methyl-1-propanol, dimethyl sulfoxide, pentane, ethanol, 1-pentanol, ethyl acetate, 1-propanol, ethyl ether, 2-propanol, ethyl formate, propyl acetate, formic acid, triethylamine, 1,1-diethoxypropane, 1,1-dimethoxymethane, 2,2-dimethoxypropane, isooctane, isopropyl ether, methylisopropyl ketone, methyltetrahydrofuran, petroleum ether, trichloroacetic acid, trifluoroacetic acid, or a combination thereof.

Preferably said further solvent is added to the solution of said first component (A) in a proportion in the range of 20-40%v/v, preferably in the range of 25-35%v/v.

According to a further preferred embodiment of the method, in the first step said first component (A) it is dissolved in a concentration in the range of 10-35% w/v, preferably in the range of 20-30% w/v, in each case per volume of the organic solvent.

According to yet another aspect of the present invention, it relates to the use of a device as defined above for the preparation of an implantable device.

According to a preferred aspect of this use, in a first step an implantable device, preferably a non-degradable implantable device (for example a pacemaker), is at least partially covered or surrounded by at least one such device, and preferably in a second step this is inserted into a wet or humid pouch of at least one of self-supporting hydrogel, cellulose or collagen through an opening thereof.

In fact, the humid pouch of at least one of self-supporting hydrogel, cellulose or collagen is non-elastic, which can preferably be quantified in that after applying and releasing tension in loading and unloading stress-strain curves with even a low stress (stress values of σ as low as 0.005, 0.01 of 0.5 MPa, or in the range of 0.01-0.1 MPa), the material of the pouch is unable to go back to its initial strain-free state.

The cellulose layer of the pouch can be provided as non-degradable pouch, therefore accompanying the non-degradable implantable device for its entire lifetime. In the alternative, the cellulose layer can be provided as degradable element which is resorbed by the body in a certain amount of time.

Key elements of this use can be as follows:
The therapeutically active substances, in the form of antibiotic shall be provided in an efficient way so as to avoid post implantation infections and the like. A cellulose pouch which is suitable and adapted to stay in place after the implantation and to remain around the non-degradable implantable device is typically produced in a biochemical process involving living organisms, so incorporating a corresponding therapeutically active substance into the cellulose pouch material is not possible, in particular not for antibiotics. Further, the cellulose pouch material cannot be prepared and then provided as a dry layer for many practical reasons, related to the storage and packaging, preparation and use. Also impregnation or coating of the pouch material after the making process with therapeutically active substance is not possible, since the wet or humid cellulose will suffer from the presence of the antibiotic or another therapeutically active substance during storage, and since the direct presence of such a material on the wet or humid cellulose pouch material will change the properties thereof, and coatings on the wet cellulose would not adhere. Further most therapeutically active substances cannot be stored under wet or humid conditions without significantly impairing storage time for degradation reasons. Moreover, soaking of the cellulose pouch in a solution containing the APIs would not result in a sustained release of the APIs, so it would not be a suitable alternative to the proposed solution.

The proposed approach therefore provides for a very simple and versatile solution to post implantation infections and the like. The proposed method is independent of the non-degradable implantable device, i.e. it can be combined with any kind of non-degradable implantable device.

The cellulose pouch is porous and open for diffusion of the therapeutically active substance through the material of the pouch. The cellulose pouch not only allows for penetration of the therapeutically active substance, it also distributes the therapeutically active substance over its surface, thereby homogenizing the release of the therapeutically active substance essentially over the full surface even if not the full inner surface of the pouch is covered by the separate layers.

Typically the bacteria causing problems in the implantation process are normally located on the surface of the implantable device. By locating the layers right on that surface the antibiotic is exactly located where it needs to act, and its dosage is highly controlled contrary to for example dip coating processes.

According to a first preferred embodiment, the wet or humid cellulose pouch has a water content in the range of 50 - 98%, preferably in the range of 90 - 98 %.

According to a further preferred embodiment, the wet or humid cellulose pouch has a diffusivity or rather a diffusion constant or mass diffusivity for the therapeutically active substance in water in the range of 10⁻¹¹ to 10⁻¹⁰ m²/s, preferably in the range of 10⁻¹⁰ to 9*10⁻¹⁰ m²/s, normally at a temperature of 37°C, wherein preferably these values are given for a cellulose pouch. In practice, the values are determined for the effective diffusivity for the therapeutically active substance (e.g. Minocycline, in particular in the form of Minocycline HCI, and Rifampicin) across a cellulose layer and measured in water at 37 degrees, as compared with deviation from free diffusion in water for the same molecules. This deviation is introduced by the porosity and tortuosity of the porous material.

The wet or humid cellulose pouch typically has a thickness of at least 0.3 mm, preferably in the range of 0.5-10 mm or in the range of 0.5-5 mm.

The wet or humid cellulose pouch may have, at least on its outside surface, a topographical surface structure with a height in the range of 0.5-2 µm, and in case of a groove/ridge topographical structure a periodicity of the structure in the range of 0.5-100 µm and in case of a pillar topographical structure a periodicity of the structure a periodicity at least in one dimension, preferably in three different directions, in the range of 5 - 50 µm, preferably in the range of 7 - 15 µm. Preferably, the topographical structure is one as described in WO2015040106A1.

Accordingly, according to a preferred embodiment the wet or humid cellulose pouch is produced before said first step using self-assembled production of a topographically surface structured cellulose element wherein a mold with on one side a first surface which is in a complementary manner topographically surface structured and which is permeable to oxygen is provided, wherein a liquid growth medium containing cellulose producing bacteria is provided, and wherein the mold is placed to form a liquid/air interface of the liquid growth medium such that the side of the mold with the first surface is in direct contact with the liquid growth medium, and with an opposite side is facing air or a specifically provided oxygen containing gas surrounding, allowing for said bacteria to produce and deposit cellulose on said first surface and developing on the interface therewith a topographically surface structured surface complementary thereto, until a contiguous cellulose layer with a thickness of the element of at least 0.3mm is formed;
and wherein in a following step the element is removed from said mold, and said pouch is produced from said element.

Preferably, between this production, which may entail actual formation of the pouch with an opening by sealing edges after a folding of a sheet, and the first step the element or the pouch can be stored in a wet environment, preferably in a corresponding suitable and adapted container.

Said non-degradable implantable device can be selected for example from the group of cardiovascular implant and/or device, in particular a pacemaker or cardioverter defibrillator; neurostimulator, neuromodulator, implantable pulse generators, cosmetic implant, preferably in the form of a breast implant, cuff implant, pectoral implant, biceps implant, buttock implant, gluteal implant; orthopedic prosthesis; a sensor and/or electrical stimulation device; draining system, preferably a catheter; pump or tubing system; ophthalmological device; hearing device; bionic device.

Subsequent to the second step in a third step said opening is typically closed, preferably by way of a suture, crimping or gluing, or a combination thereof wherein elements, in particular tubing and/or wiring attached to and connected with said non-degradable implantable device if present remain penetrating said opening.

Further the present invention relates to a kit of parts for use in a method as described above, comprising
at least one, preferably wet or humid, pouch, preferably in the form of a pouch of at least one of self-supporting hydrogel, cellulose or collagen, having an opening in a first wet package;
and at least one device as given above in a separate wet or dry package, preferably in a dry package.

There is preferably provided at least two devices in such a separate package, and preferably the devices take the form of rectangular or quadratic sheets with length and/or width in the range of 50-110 mm, preferably in the range of 60-90 mm.

Preferably they take the form of a rectangle having a length in the range of 70-110 mm, preferably in the range of 80-90 mm, and a width in the range of 50-90 mm, preferably in the range of 60-80 mm.

Preferably said first package comprises elements for maintaining controlled humidity, preferably in the form of aluminium or aluminium plastic laminate pouches, and/or said separate package comprises elements for maintaining dryness, preferably desiccant elements.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

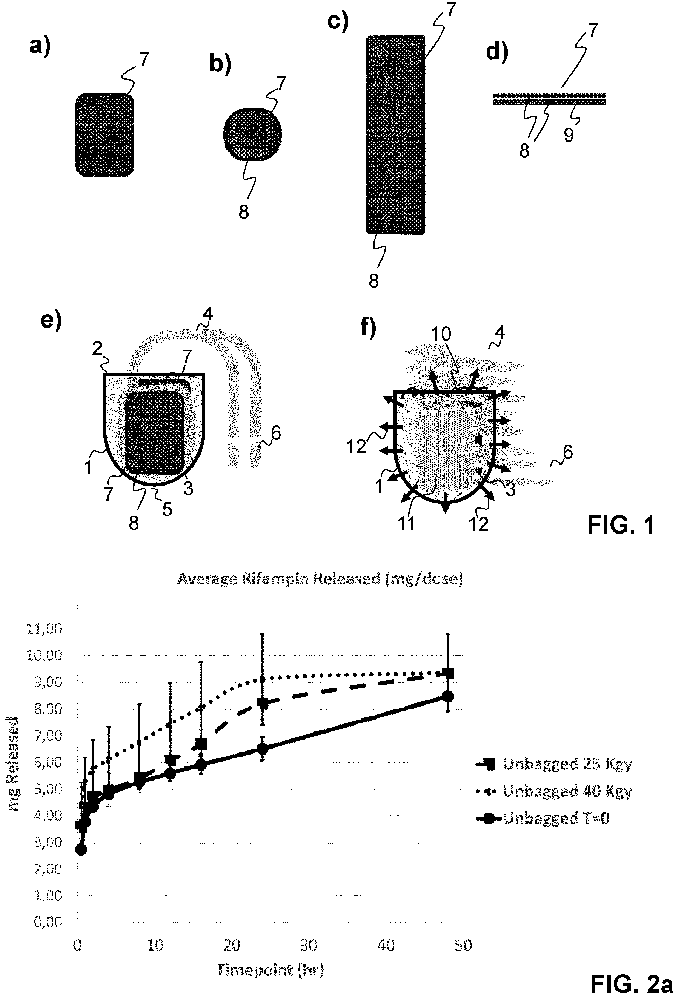

- Fig. 1: shows in a)-c) top views onto differently shaped devices as proposed and in d) a side view onto such a device in a multilayer design, in e) a non-degradable implantable device with two separate devices inserted into the pouch; in f) a schematic representation of the release of the therapeutically active substance out of the implantable device;

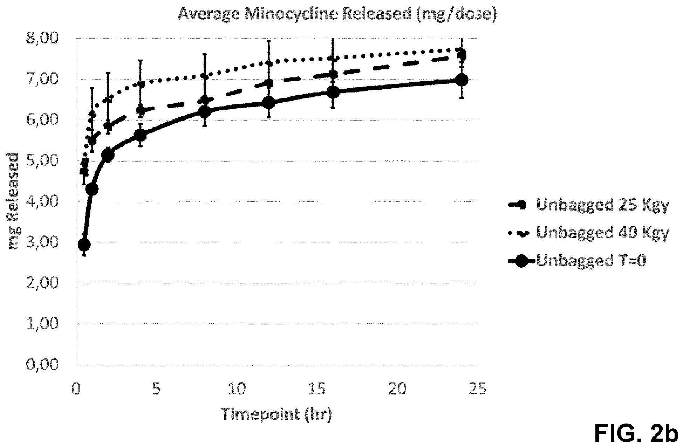

- Fig. 2: shows in a) shows the elution profile for Rifampicin (average of API released in milligrams/dose) before and after sterilization with irradiation at 25 or 40 Kgy and in b) the elution profile for Minocycline (average of API released in milligrams/dose) before and after sterilization with irradiation at 25 or 40 Kgy.

### DESCRIPTION OF PREFERRED EMBODIMENTS

In Fig. 1 schematically a device 7 is shown and different shapes thereof are illustrated in Figs. a)-c). In case of these figures the device consists of one single layer 8 of the matrix with the API as defined above.

As illustrated in a side view in Fig. 1d), the device 7 may comprise more than one layer 8, for example it may have a central support layer 9, which then is also preferably biodegradable, and the two layers 8 of the matrix with the API are provided on both sides of that support layer 9. The support layer 9 can also take the form of a grid or mesh, while the layers 8 typically are contiguous layers.

**Fig. 1e**) and **f)** schematically show a possible use of such a device using an example of a pacemaker. A cellulose pouch 1 is provided in a wet or humid form as a separate element. It is typically taken out of a package providing for wet storage by the corresponding personnel. Tools can be used for handing these cellulose pouches, to simplify the procedure. The pouch is based on a patch which is typically folded over one edge and has a sealed edge 5 and only one opening 2 thereby forming the actual pouch for insertion of the non-degradable implantable device.

Separately the non-degradable implantable device 3 is provided. In this case it is a pacemaker having an actual core element with front surface and back surface, so it is a flat almost rectangular device. It further comprises wiring 4 which may be attached to an external device 6. However the proposed method may also be carried out with implantable device which do not have wiring or tubing to the outside, such as for example breast implants or the like.

Before inserting, the front surface and the back surface of the non-degradable implantable device 3 is covered by controlled release devices or pads 7. These controlled release pads 7 are provided as separate elements and are typically unpacked from corresponding dry storage packages. As mentioned above, typically pouch and these controlled release pads are provided in separate packages or in separate compartments of one larger package as a kit of parts for ease of use. The making of these release pads 7 will be detailed further below. They may manually be arranged in contact with and facing the front and back surface of the non-degradable implantable device, and this may be assisted, in particular in case of very thin dry controlled release pads 7 by electrostatic attraction between the device 3 and the respective sheets of the release pads 7.

This assembly of the non-degradable implantable device 3 and the 2 controlled release pads 7 is now taken and inserted into the pouch 1 through the opening, and the result of that process is illustrated in **Fig. 1e**). After that typically the opening is closed, in this case by way of a suture 10.

This prepared implantable device is then, subsequent to this preparation method, implanted into the human body. Under the conditions in the human body (temperature, physiological liquids) the therapeutically active substances start diffusing/migrating through the porous cellulose patch material into the surrounding body tissue. If the therapeutically active substance is an antibiotic, it is then accordingly, over a defined period of time (sustained-release) released into that portion of the tissue where the risk of an infection is highest. So it is a very targeted selective release of the active agent exactly to the place and over the time as needed. Correspondingly an as low as possible dosage of an antibiotic can be used.

In this process, which is illustrated in **Fig. 1f****),** the sheets 7 either just release the active ingredient, or there is a whole, if they are degradable, or dissolvable, at the same time as releasing the active ingredient start to disintegrate (partially degraded element illustrated as 11) and, after a certain time, vanish completely. Until then the release 12 of the active ingredient takes place.

To evidence the inventiveness of the present invention, it shall be described how and in which context the finally claimed subject matter, in particular the specific polymer blend selection for the matrix was developed.

### FIRST PHASE:

In the first selection phase several different polymers were evaluated. The design boundaries were the following:
   - a combination of water-soluble and water insoluble therapeutically active substances should be released in a controlled way, in particular APIs - Minocycline which is water soluble - Rifampicin which is water insoluble as example systems .
      ∘ Rifampicin (7.6 mg) and Minocycline (11.9 mg) per film homogenous inclusion.
      ∘ Elution profile.
   - Usability
      ∘ Flexible not brittle
      ∘ No sharp edges (any shape possible)
      ∘ Thin (any thickness possible)
      ∘ Slightly adhesive (to easily adhere to the target implant - but also to be removable if needed)
   - Shelf life - more than 1 year
   - Sterilization - Possible with irradiation and/or Et-Oxide

The following polymers were tested with a note on the results:
• Polyvinyl alcohol (PVA) - was extensively tested, however had to be discarded for its fast dissolution in water. The excipient is therefore not suited for a sustained release application.
• Hydroxypropyl methylcellulose (HPMC) - The testing was rapidly terminated due to the high amount of water absorbed by HPMC and the formation of a hydrogel. This contrasts with the low water solubility of Rifampicin.
• Silicone - Preliminary tests performed leading to a homogeneous film. However, no suitable implant grade silicon was available.
• Ethylene-vinyl acetate (EVA) - was extensively tested and optimized. However, EVA proved non-biodegradable and this option was discarded.
• Polylactic Acid (PLA) - Polylactic-co-glycolic acid (PLGA) - A formulation leading to good film was developed. Adding PEG enabled the formation with a film with good elasticity. In the initial formulations the dissolution profiles of the two APIs were much slower than the intended design. Therefore, this solution required optimization.
• Polyurethane (PU) - A good candidate to obtain a homogenous film with both APIs. However, PU is non-biodegradable, and this option was discarded.

CONCLUSIONS of the first selection phase: A procedure for the generation of biodegradable PLA/PLGA based polymers could be developed by adding PEG as plasticizer.

However, a second phase of optimization was necessary to solve the following challenges:
1. The APIs dissolution was incomplete reaching a plateau before the full amount is released. APIs seem to be trapped in the polymer.
2. Film resulted too brittle and difficult to peel from the backing foil without ripping.

### SECOND PHASE:

The main goals are summarized below:
1. Prepare placebo films with multiple grades of PLGA polymers (Table 1) using solvent casting method. Evaluate both single and dual layer film designs.
2. Evaluate solubility of Rifampicin and Minocycline in several solvents and determine ideal solvent for film formulation.
3. Introduce Rifampicin and Minocycline to placebo formulations and evaluate film properties after APIs addition.
4. Create HPLC assay and drug elution method based on provided information. Optimize method for use in formulation development.

**Table 1: Identified PLGA polymer samples**

| Supplier | Polymer Name | PLGA Ratio | Inherent Viscosity (molecular weight) |
|---|---|---|---|
| Corbion | Purasorb PDLG 1 | 50:50 | 1.0 |
| | Purasorb PDLG 2 | 75:25 | 0.7 |
| Evonik | Resomer RG 505 | 50:50 | 0.6 |
| | Resomer RG 502 H | 50:50 | 0.2 |
| | Resomer RG 750 S | 75:25 | 1.0 |

Using these test materials the following results were obtained:
The PDLG 1 material (molecular weight (number average) 60'000+/-6'000 g/mol, molecular weight (weight average) 125'000+/-10'000 g/mol, glass transition temperature 48.5°C) with a solids content of 15% and 3.0 g of polymer added lead to an ideal viscosity, formation of a gel over time, and providing the possibility of reprocessing. The film thickness was 0.42 mm and the film properties: malleable, low tensile stretchy, but still significant air bubble formation during coating due to skinning.

The PDLG 2 material (molecular weight (number average) 50'000+/-2'000 g/mol, molecular weight (weight average) 90'000+/-3'000 g/mol, glass transition temperature 49.5°C) with a solids content of 21% and 4.0 g of polymer added lead to too low viscosity but a uniform coat, the film thickness was about 0.34 mm. The layer was malleable, soft to touch and easy to compress. Tensile strength was insufficient.

The Resomer 505 material (molecular weight (number average) 50'000+/-1'000 g/mol, molecular weight (weight average) 80'000+/-2'000 g/mol, glass transition temperature 48.2°C) with the same solids content and polymer added lead to too low viscosity and a considerable flow during coating. Tensile strength too low.

The Resomer 750 material with 3.0 g solids content and 15% polymer added lead to too low viscosity and low tensile strength layer.

### Results phase 2

Active coat feasibility. The following procedures were tested,
- API then polymer 'DIP COAT'
- Polymer and API 'DIP COAT'
- Drug in suspension 'solvent casting'

### Conclusions:

• Minocycline HCI is not soluble in most PLGA solvents (THF, Acetone, Ethyl Acetate) but highly soluble in Methanol. Use of dichloromethane (DCM) may be alternate approach.
• Films with suitable physical properties can be made from PLGA by both solvent casting and dip coating methods.
• Increased drug elution is observed in formulations with higher drug to polymer ratio (50:50 vs 80:20).
• Suspended drug (Minocycline) appears to elute faster from film matrix than dissolved drug (Rifampicin).

Based on these results two formulations were tested:
• Formulation 1 - One thin film of PLGA with both APIs.
• Formulation 2 - Two separate film layers each containing one API.

Results are reported in the figures below for visual appearance and elution profile as compared to a predicate device.

### Conclusion:

• Significant degradation of Minocycline HCI occurred during elution testing of all prototypes. Investigation/optimization needed to obtain accurate elution testing results for Minocycline HCI.
• Formulation 1 does not match desired in-vitro release properties. The amount of suspended drug will need to be reduced.
• Formulation 2 (two separate thin films combined to make final product) Rifampicin elution results were lower than the predicate device (TYRX) at all timepoints. The elution profile curve however closely matches predicate device. Further optimization of this formulation is needed to match in-vitro release properties of TYRX.

### THIRD PHASE:

Film optimization. Multiple aspects required optimization:
• Avoid degradation of minocycline into epiminocycline.
• Use of PEG-4000 plasticizer and to have effect on API degradation.
• Film appearance, mechanics, coat weight and drug stability.

### Results:

• Film thickness has rather low impact on drug elution for either API.
• Formulations with PEG-4000 have a different elution profile (lower initial drug release).
• Elution profile is promising and evaluated in cellulose bags and when films are exposed to sterilization.
• Drug layer will need to be coated on PGA substrate to evaluate impact of substrate to early timepoint release.
• PEG-400o enhances API degradation if used outside of the good proportions

These results led to the final formulation which is identified by the following:

### Film Composition Considerations:

• Two Antibiotics- Minocycline HCI (7.6 mg per dose), Rifampicin (11.9 mg per dose)
• PLGA - copolymer of DL-lactide and Glycolide in a 50/50 molar ratio and with an inherent viscosity midpoint of 1.0 dl/g
• Single Layer Feasible at higher coat weight with plasticizer (PEG 4000)
• Dual layer possible to reduce coat variability/curling (extruding of PGA material) Physical Characteristics:
• Film Size- 15 cm² (~3x5 cm)
• Film Thickness- Provides required physical characteristics
• Adheres to implantable device
• Does not tear or stretch when removed from liner
• Can be applied to pacemaker using forceps
• Holds shape when inserted to cellulose bag.

### Elution Profile:

• Extended linear release of both APIs was seen for drug elution testing. Rifampicin eluted at a slower more linear rate than Minocycline HCI.
• Introduction of cellulose envelopes to in vitro testing slowed the drug elution.
• Sterilization does not change results (Fig. 2).

### Making of an optimized pad:

Table 2 summarizes the formulation and materials used for mixing. One roll of release liner was used. Paper cores were used as a substitute for plastic cores.

**Table 2: mixing Materials and Formulation**

| | **Excipient** | | **Theoretical Percent wt./v (%)** |
|---|---|---|---|
| | | | **A** |
| | Acetonitrile | | 48 |
| | PURASORB PDLG 1 | | 17 |
| | Methanol | | 21 |
| | Minocycline Hydrochloride | | 5 |
| | Rifampicin | | 6 |
| | Polyethylene Glycol 4000 | | 3 |
| **Total** | | | 100.0 |

The blend was mixed and a mixer was used to provide agitation. To avoid solvent evaporation during mixing, the bottle was capped.

Coating was performed on Pilot Coater. The knife over roll coating technique was used to coat the blend onto release liner. To minimize losses, the blend was added to coating head manually via sampling thief.

### 1.3 Process Summary Results

Mixing and coating was performed in temperature and humidity controlled cleanroom environment .

The mixing process followed this sequence:
1. Acetonitrile.
2. PLGA.
3. Methanol.
4. Minocycline HCI, Rifampicin, and PEG.
5. The mixing container was degassed.

All mixing steps (1-5) were performed at room temperature.

After mixing, the blend was held under vacuum. The vessel was then sealed and held under atmospheric pressure after sampling.

The coating process is as follows:
1. The Pilot Coater was webbed.
2. The dryer sections of the Pilot Coater were started.3.
3. The coating gap was set to desired coating thickness.
4. Once dryer sections reached target temperature, the coating line was started.
5. A portion of the blend was manually delivered to the coating setup.
6. The blend was coated out. The resulting laminate was wound on paper cores in roll form coming out of the dryer sections.

Preliminary process parameters for coating and drying process were initially defined during formulation development / prototyping stage of the project and were successfully trialed during informal placebo batch manufacturing. These preliminary CPPs are summarized in Table 4.

**Table 4: Coating Process Parameters**

| **Parameter** | **Value** | **Parameter** | **Value** |
|---|---|---|---|
| Dryer Temp. (Zone 1) | 40 - 65 °C | Coating Gap | 0.03 - 0.2 mm |
| Dryer Air Supply (Zone 1) | 60-80% | Unwind Tension | 30 - 50 N |
| Dryer Air Exhaust (Zone 1) | 70-90% | Rewind Tension | 30 - 50 N |
| Dryer Temp. (Zone 2) | 50 - 80 °C | Coater Tension | 25 - 45 N |
| Dryer Air Supply (Zone 2) | 60 - 80% | Damper | 80 - 100% |
| Dryer Air Exhaust (Zone 2) | 60 - 80% | Line Speed | 0.1 - 1 m/min |

### LIST OF REFERENCE SIGNS

| | | | |
|---|---|---|---|
| 1 | cellulose pouch | | pad |
| 2 | opening of 1 | 8 | outer layer |
| 3 | non-degradable implantable device | 9 | intermediate layer |
| | | 10 | suture |
| 4 | wiring/tubing to 3 | 11 | partially degraded/dissolved |
| 5 | sealed edge of 1 | | 7 |
| 6 | external device of 3 | 12 | release of active agent, antibiotic |
| 7 | device, controlled release | | |

## Claims

1. Device (7) for sustained controlled release, of at least two therapeutically active substances after implantation of said device alone or in combination with further implantable elements (1,3), said device (7) comprising at least one layer (8) of material comprising at least two therapeutically active substances and providing for sustained controlled release of said at least two substances under physiological conditions,
wherein said layer (8) comprises a biodegradable matrix consisting of a mixture of
| | |
|---|---|
| 75-95% | by dry weight of a first component (A) in the form of a copolymer of DL-lactide and glycolide (PLGA) with a molar ratio of DL-lactide to glycolide in the range of 45:55-55:45and with a weight average molecular weight (MW_{w}) in the range of 100,000-150,000 g/mol and |
| 5-25% | by dry weight of a second component (B) in the form of polyethylene glycol (PEG) with a weight average molecular weight (MW_{w}) in the range of 3000-5500 g/mol, |
the dry weights of component (A) and (B) complementing to 100% of said matrix,
wherein in this matrix the at least two therapeutically active substances are dissolved and/or suspended
wherein of the two therapeutically active substances one is hydrophobic with a solubility of less than 5 mg/ml and one is hydrophilic with a solubility of more than 20 mg/ml, in each case measured at pH 7 and at room temperature of 20 deg,
wherein the at least two therapeutically active substances are selected from at least two antibiotics selected from the group of tetracyclines, penicillins, macrolides, ansamycines, and wherein the total of therapeutically active substances in the layer (8) is in the range of 25-40% given as %w/w dry with respect to the total of the layer (8).

2. Device (7) according to claim 1, wherein said first component (A) has a molar ratio of DL-lactide to glycolide in the range of 48:52-52:48, or in the range of about 50:50.

3. Device (7) according to any of the preceding claims, wherein said first component (A) has a weight average molecular weight (MW_{w}) in the range of 110,000-140,000 g/mol, preferably in the range of 120,000-130,000 g/mol,
and/or wherein said first component (A) has a number average molecular weight (MWₙ) in the range of 50,000-100,000 g/mol, preferably in the range of 60,000-80,000 g/mol, most preferably in the range of 65,000-75,000 g/mol,
and/or wherein said first component (A) has an inherent viscosity midpoint in the range of 0.8-1.2 dl/g, preferably in the range of 0.9-1.1 dl/g (in each case measured in chloroform, 25 °C, c = 0.1 g/dl).

4. Device (7) according to any of the preceding claims, wherein said second component (B) has a weight average molecular weight (MW_{w}) in the range of 3200-4800 g/mol, most preferably in the range of 3700-4400 g/mol,
and/or wherein said second component (B) has a solidification point in the range of 45-68°C, preferably in the range of 50-65°C, most preferably in the range of 55-60°C.

5. Device (7) according to any of the preceding claims, wherein said layer (8) comprises a matrix consisting of a mixture of
75-92% by dry weight, preferably 80-90% by dry weight, most preferably 84-88% by dry weight of component (A) and
8-25% by dry weight, preferably 10-20% by dry weight, most preferably 12-16% by dry weight of component (B)
the dry weights of component (A) and (B) complementing to 100% of said matrix.

6. Device (7) according to any of the preceding claims, wherein the at least two therapeutically active substances are selected from the group consisting of Tetracycline, Chlortetracycline, Oxytetracycline, Demeclocycline, Lymecycline, Meclocycline, Methacycline, Minocycline, in particular in the form of Minocycline HCI, Rolitetracycline, Doxycycline, Tigecycline, Eravacycline, Sarecycline, Omadacycline, Rifampicine.

7. Device (7) according to claim 6, wherein the at least two therapeutically active substances are a combination of Minocycline, in particular in the form of Minocycline HCI, and Rifampicine.

8. Device (7) according to any of the preceding claims, wherein the layer (8) as at least two therapeutically active substances comprises a combination of Minocycline, in particular in the form of Minocycline HCI, and Rifampicin in a total concentration of 25-40% by weight, preferably in the range of 25-35% by weight, in each case given as %w/w dry with respect to the total of the layer,
wherein preferably the concentration of Minocycline, in particular in the form of Minocycline HCI, is in the range of 10-20% by weight, more preferably in the range of 14-18%,
and/or wherein the concentration of Rifampicin is in the range of 15-25% by weight, preferably in the range of 17-22% by weight, in each case given as %w/w dry with respect to the total of the layer.

9. Device (7) according to any of the preceding claims, wherein said layer (8) comprises a matrix consisting of a mixture of
84-88% by dry weight of first component (A) with a weight average molecular weight in the range of 120,000-130,000 g/mol,
and
12-16% by dry weight of second component (B) with a weight average molecular weight MWw in the range of 3700-4400 g/mol,
the dry weights of component (A) and (B) complementing to 100% of said layer (8),
and wherein the layer (8) as at least two therapeutically active substances comprise a combination of Minocycline, in particular in the form of Minocycline HCI and Rifampicine in a total concentration of 25-35% by weight,
wherein the concentration of Minocycline, in particular in the form of Minocycline HCI is in the range of 14-18%, and the concentration of Rifampicine is in the range of 17-22% by weight,
in each case given as %w/w dry with respect to the total of the layer.

10. Device (7) according to any of the preceding claims, wherein it takes the form of at least one or only one self-supporting film (8), mesh, non-woven structure or combination thereof, preferably having a thickness in the range of 2.5-100 µm, preferably in the range of 10-30 µm,
wherein it further preferably takes the form of a film or patch having a maximum extension in a lateral direction of 30 cm, preferably an extension in the range of 0.5-15 cm, more preferably in the range of 1-10 cm,
wherein further preferably the film or patch takes a round, oval or polygonal shape, preferably a rectangular or square shape, optionally with rounded edges, and
and/or wherein the device may consist of one single or more than one of said layers (8), in combination with at least one additional supporting layer (9), or most preferably the device (7) consists of one single layer (8).

11. Method for making a device (7) according to any of the preceding claims, wherein
in a first step said first component (A) is fully dissolved in an organic solvent, preferably selected from the group of acetonitrile, toluene, anisole, chloroform, dichloromethane, dimethylformamide, dimethylsulfoxide, ethyl acetate, dioxane, tetrahydrofuran, toluene, or a combination thereof,
preferably under stirring at a temperature in the range of 15-30°C, preferably at room temperature (20-25°C),
to firm a solution of said first component (A),
and wherein
in a second step said second component (B) and the at least two therapeutically active substances are added to said solution of said first component (A),
preferably under stirring at a temperature in the range of 15-30°C, preferably at room temperature,
followed by evaporation of the at least one solvent to form the device, preferably in a solution casting or solution coating process, preferably onto a release material.

12. Method according to the preceding claim, wherein after said first step and before carrying out said second step, a further organic solvent, different from the organic solvent used in the first step, is added to the solution of said first component (A), wherein preferably said further organic solvent is selected from the group of acetonitrile, chlorobenzene, chloroform, cumene, cyclohexane, 1,2-dichloroethene, dichloromethane,1,2-dimethoxyethane, N,N-dimethylacetamide, N,N-dimethylformamide, 1,4-dioxane, 2-ethoxyethanol, ethyleneglycol, formamide, hexane, methanol, 2-methoxyethanol, methylbutyl ketone, methylcyclohexane, methylisobutylketone, N-methylpyrrolidone, nitromethane, pyridine, sulfolane, tetrahydrofuran, tetralin, toluene, 1,1,2-trichloroethene, xylene, acetic acid, heptane, acetone, isobutyl acetate, anisole, isopropyl acetate, 1-butanol, methyl acetate, 2-butanol, 3-methyl-1-butanol, butyl acetate, methylethyl ketone, tert-butylmethyl ether, 2-methyl-1-propanol, dimethyl sulfoxide, pentane, ethanol, 1-pentanol, ethyl acetate, 1-propanol, ethyl ether, 2-propanol, ethyl formate, propyl acetate, formic acid, triethylamine, 1,1-diethoxypropane, 1,1-dimethoxymethane, 2,2-dimethoxypropane, isooctane, isopropyl ether, methylisopropyl ketone, methyltetrahydrofuran, petroleum ether, trichloroacetic acid, trifluoroacetic acid, or a combination thereof,
wherein preferably said further solvent is added to the solution of said first component (A) in a proportion in the range of 20-40%v/v, preferably in the range of 25-35%v/v,
and/or wherein in the first step said first component (A) it is dissolved in a concentration in the range of 10-35% w/v, preferably in the range of 20-30% w/v, in each case per volume of the organic solvent.

13. Use of a device (7) according to any of the preceding claims 1-10 for the preparation of an implantable device,
wherein in a first step an implantable device (3), preferably a non-degradable implantable device (3), is at least partially covered or surrounded by at least one such device (7),
and wherein preferably in a second step this is inserted into a wet or humid pouch (1) of at least one of self-supporting hydrogel, cellulose or collagen (1) through an opening (2) thereof.

14. Kit of parts for a use according to the preceding claim, comprising
at least one wet or humid pouch (1), preferably in the form of a pouch of at least one of self-supporting hydrogel, cellulose or collagen (1), having an opening (2) in a first wet package;
and at least one device according to any of the preceding claims 1-10 (7) in a separate wet or dry package, preferably in a dry package.

15. Kit of parts according to claim 14, wherein there is provided at least two devices (7) in said separate package, and wherein preferably the devices take the form of rectangular or quadratic sheets with length and/or width in the range of 50-110 mm, preferably in the range of 60-90 mm, wherein they preferably take the form of a rectangle having a length in the range of 70-110 mm, preferably in the range of 80-90 mm, and a width in the range of 50-90 mm, preferably in the range of 60-80 mm
and/or wherein preferably said first package comprises elements for maintaining controlled humidity, preferably in the form of aluminium or aluminium plastic laminate pouches,
and/or wherein said separate package comprises elements for maintaining dryness, preferably desiccant elements.

## Patentansprüche

1. Vorrichtung (7) zur verzögerten kontrollierten Freisetzung von mindestens zwei therapeutisch wirksamen Substanzen nach Implantation der Vorrichtung allein oder in Kombination mit weiteren implantierbaren Elementen (1, 3), wobei die Vorrichtung (7) mindestens eine Schicht (8) aus einem Material umfasst, das mindestens zwei therapeutisch wirksame Substanzen enthält und unter physiologischen Bedingungen für eine verzögerte kontrollierte Freisetzung der mindestens zwei Substanzen sorgt,
wobei die Schicht (8) eine biologisch abbaubare Matrix umfasst, die aus einer Mischung von
| | |
|---|---|
| 75-95 %, | bezogen auf das Trockengewicht, einer ersten Komponente (A) in Form eines Copolymers aus DL-Lactid und Glycolid (PLGA) mit einem Molverhältnis von DL-Lactid zu Glycolid im Bereich von 45:55 bis 55:45 und mit einem gewichtsmittleren Molekulargewicht (MW_{w}) im Bereich von 100.000-150.000 g/mol und |
| 5-25 %, | bezogen auf das Trockengewicht, einer zweiten Komponente (B) in Form von Polyethylenglykol (PEG) mit einem gewichtsmittleren Molekulargewicht (MW_{w} ) im Bereich von 3000-5500 g/mol, |
wobei die Trockengewichte der Komponenten (A) und (B) zusammen 100 % der Matrix ergeben,
wobei in dieser Matrix die mindestens zwei therapeutisch wirksamen Substanzen gelöst und/oder suspendiert sind
wobei von den beiden therapeutisch wirksamen Substanzen eine hydrophob mit einer Löslichkeit von weniger als 5 mg/ml und eine hydrophil mit einer Löslichkeit von mehr als 20 mg/ml ist, jeweils gemessen bei pH 7 und bei einer Raumtemperatur von 20 °C,
wobei die mindestens zwei therapeutisch wirksamen Substanzen aus mindestens zwei Antibiotika ausgewählt sind, die aus der Gruppe der Tetracycline, Penicilline, Makrolide und Ansamycine ausgewählt sind, und wobei die Gesamtmenge der therapeutisch wirksamen Substanzen in der Schicht (8) im Bereich von 25 bis 40 % liegt, angegeben als Trockengewichtsprozent %w/w in Bezug auf die Gesamtmenge der Schicht (8).

2. Vorrichtung (7) nach Anspruch 1, wobei die erste Komponente (A) ein Molverhältnis von DL-Lactid zu Glycolid im Bereich von 48:52 bis 52:48 oder im Bereich von etwa 50:50 aufweist.

3. Vorrichtung (7) nach einem der vorstehenden Ansprüche, wobei die erste Komponente (A) ein gewichtsmittleres Molekulargewicht (MW_{w} ) im Bereich von 110.000 bis 140.000 g/mol, vorzugsweise im Bereich von 120.000 bis 130.000 g/mol, aufweist
und/oder wobei die erste Komponente (A) ein zahlenmittleres Molekulargewicht (MWₙ ) im Bereich von 50.000 bis 100.000 g/mol, vorzugsweise im Bereich von 60.000 bis 80.000 g/mol, am bevorzugtesten im Bereich von 65.000 bis 75.000 g/mol aufweist,
und/oder wobei die erste Komponente (A) einen mittleren spezifischen Viskositätswert im Bereich von 0,8 bis 1,2 dl/g, vorzugsweise im Bereich von 0,9 bis 1,1 dl/g (jeweils gemessen in Chloroform, 25 °C, c = 0,1 g/dl) aufweist.

4. Vorrichtung (7) nach einem der vorstehenden Ansprüche, wobei die zweite Komponente (B) ein gewichtsmittleres Molekulargewicht (MW_{w} ) im Bereich von 3200 bis 4800 g/mol, am bevorzugtesten im Bereich von 3700 bis 4400 g/mol, aufweist,
und/oder wobei die zweite Komponente (B) einen Erstarrungspunkt im Bereich von 45 bis 68 °C, vorzugsweise im Bereich von 50 bis 65 °C, am bevorzugtesten im Bereich von 55 bis 60 °C aufweist.

5. Vorrichtung (7) nach einem der vorstehenden Ansprüche, wobei die Schicht (8) eine Matrix umfasst, die aus einer Mischung aus
75 bis 92 Gew.-%, vorzugsweise 80 bis 90 Gew.-%, am bevorzugtesten 84 bis 88 Gew.-% der Komponente (A) und
8 bis 25 Gew.-%, vorzugsweise 10 bis 20 Gew.-%, am bevorzugtesten 12 bis 16 Gew.-% der Komponente (B)
wobei die Trockengewichte der Komponenten (A) und (B) zusammen 100 % der Matrix ergeben.

6. Vorrichtung (7) nach einem der vorstehenden Ansprüche, wobei die mindestens zwei therapeutisch wirksamen Substanzen ausgewählt sind aus der Gruppe bestehend aus Tetracyclin, Chlortetracyclin, Oxytetracyclin, Demeclocyclin, Lymecyclin, Meclocyclin, Methacyclin, Minocyclin, insbesondere in Form von Minocyclin-HCl, Rolitetracyclin, Doxycyclin, Tigecyclin, Eravacyclin, Sarecyclin, Omadacyclin, Rifampicin.

7. Vorrichtung (7) nach Anspruch 6, wobei die mindestens zwei therapeutisch wirksamen Substanzen eine Kombination aus Minocyclin, insbesondere in Form von Minocyclin-HCl, und Rifampicin sind.

8. Vorrichtung (7) nach einem der vorstehenden Ansprüche, wobei die Schicht (8) als mindestens zwei therapeutisch wirksame Substanzen eine Kombination aus Minocyclin, insbesondere in Form von Minocyclin-HCl, und Rifampicin in einer Gesamtkonzentration von 25 bis 40 Gew.-%, vorzugsweise im Bereich von 25 bis 35 Gew.-%, jeweils angegeben als % w/w trocken bezogen auf die Gesamtmenge der Schicht,
wobei vorzugsweise die Konzentration von Minocyclin, insbesondere in Form von Minocyclin-HCl, im Bereich von 10 bis 20 Gew.-%, noch bevorzugter im Bereich von 14 bis 18 Gew.-%, liegt,
und/oder wobei die Konzentration von Rifampicin im Bereich von 15 bis 25 Gew.-%, vorzugsweise im Bereich von 17 bis 22 Gew.-%, jeweils angegeben als %w/w trocken, bezogen auf die Gesamtmenge der Schicht, liegt.

9. Vorrichtung (7) nach einem der vorstehenden Ansprüche, wobei die Schicht (8) eine Matrix umfasst, die aus einer Mischung aus
84 bis 88 Gew.-% Trockengewicht einer ersten Komponente (A) mit einem gewichtsmittleren Molekulargewicht im Bereich von 120.000 bis 130.000 g/mol,
und
12 bis 16 Gew.-% Trockengewicht einer zweiten Komponente (B) mit einem gewichtsmittleren Molekulargewicht MWw im Bereich von 3700 bis 4400 g/mol,
wobei die Trockengewichte der Komponenten (A) und (B) zusammen 100 % der Schicht (8) ergeben,
und wobei die Schicht (8) als mindestens zwei therapeutisch wirksame Substanzen eine Kombination aus Minocyclin, insbesondere in Form von Minocyclin-HCl, und Rifampicin in einer Gesamtkonzentration von 25 bis 35 Gew.-% umfasst,
wobei die Konzentration von Minocyclin, insbesondere in Form von Minocyclin-HCI, im Bereich von 14 bis 18 % liegt und die Konzentration von Rifampicin im Bereich von 17 bis 22 Gew.-% liegt,
jeweils angegeben als Gew.-% Trockengewicht bezogen auf die Gesamtschicht.

10. Vorrichtung (7) nach einem der vorstehenden Ansprüche, wobei sie die Form von mindestens einer oder nur einer selbsttragenden Folie (8), einem Netz, einer Vliesstruktur oder einer Kombination davon hat, vorzugsweise mit einer Dicke im Bereich von 2,5 bis 100 µm, vorzugsweise im Bereich von 10 bis 30 µm,
wobei sie ferner vorzugsweise die Form einer Folie oder eines Pflasters mit einer maximalen Ausdehnung in seitlicher Richtung von 30 cm, vorzugsweise einer Ausdehnung im Bereich von 0,5 bis 15 cm, noch bevorzugter im Bereich von 1 bis 10 cm, aufweist,
wobei die Folie oder das Pflaster vorzugsweise eine runde, ovale oder polygonale Form, vorzugsweise eine rechteckige oder quadratische Form, optional mit abgerundeten Kanten, aufweist, und
und/oder wobei die Vorrichtung aus einer einzigen oder mehreren der genannten Schichten (8) in Kombination mit mindestens einer zusätzlichen Trägerschicht (9) bestehen kann, oder am bevorzugtesten besteht die Vorrichtung (7) aus einer einzigen Schicht (8).

11. Verfahren zur Herstellung einer Vorrichtung (7) gemäß einem der vorstehenden Ansprüche, wobei
**In** einem ersten Schritt wird die erste Komponente (A) vollständig in einem organischen Lösungsmittel, vorzugsweise einem aus der Gruppe Acetonitril, Toluol, Anisol, Chloroform, Dichlormethan, Dimethylformamid, Dimethylsulfoxid, Ethylacetat, Dioxan, Tetrahydrofuran, Toluol oder einer Kombination davon ausgewähl ,
vorzugsweise unter Rühren bei einer Temperatur im Bereich von 15 bis 30 °C, vorzugsweise bei Raumtemperatur (20 bis 25 °C),
um eine Lösung der ersten Komponente (A) zu erhalten,
und wobei
in einem zweiten Schritt die zweite Komponente (B) und die mindestens zwei therapeutisch wirksamen Substanzen zu der Lösung der ersten Komponente (A) hinzugefügt werden,
vorzugsweise unter Rühren bei einer Temperatur im Bereich von 15 bis 30 °C, vorzugsweise bei Raumtemperatur,
gefolgt von der Verdampfung des mindestens einen Lösungsmittels, um die Vorrichtung zu bilden, vorzugsweise in einem Lösungsguss- oder Lösungsbeschichtungsverfahren, vorzugsweise auf ein Trennmaterial.

12. Verfahren nach dem vorstehenden Anspruch, wobei nach dem ersten Schritt und vor der Durchführung des zweiten Schritts ein weiteres organisches Lösungsmittel, das sich von dem im ersten Schritt verwendeten organischen Lösungsmittel unterscheidet, zu der Lösung der ersten Komponente (A) hinzugefügt wird, wobei das weitere organische Lösungsmittel vorzugsweise aus der Gruppe ausgewählt ist, die aus Acetonitril, Chlorbenzol, Chloroform, Cumol, Cyclohexan, 1,2-Dichlorethen, Dichlormethan, 1,2-Dimethoxyethan, N,N-Dimethylacetamid, N,N-Dimethylformamid, 1,4-Dioxan, 2-Ethoxyethanol, Ethylenglykol, Formamid, Hexan, Methanol, 2-Methoxyethanol, Methylbutylketon, Methylcyclohexan, Methylisobutylketon, N-Methylpyrrolidon, Nitromethan, Pyridin, Sulfolan, Tetrahydrofuran, Tetralin, Toluol, 1,1,2-Trichlorethen, Xylol, Essigsäure, Heptan, Aceton, Isobutylacetat, Anisol, Isopropylacetat, 1-Butanol, Methylacetat, 2-Butanol, 3-Methyl-1-butanol, Butylacetat, Methylethylketon, tert-Butylmethylether, 2-Methyl-1-propanol, Dimethylsulfoxid, Pentan, Ethanol, 1-Pentanol, Ethylacetat, 1-Propanol, Ethylether, 2-Propanol, Ethylformiat, Propylacetat, Ameisensäure, Triethylamin, 1,1-Diethoxypropan, 1,1-Dimethoxymethan, 2,2-Dimethoxypropan, Isooctan, Isopropylether, Methylisopropylketon, Methyltetrahydrofuran, Petrolether, Trichloressigsäure, Trifluoressigsäure oder eine Kombination davon,
wobei vorzugsweise das weitere Lösungsmittel der Lösung der ersten Komponente (A) in einem Anteil im Bereich von 20 bis 40 % v/v, vorzugsweise im Bereich von 25 bis 35 % v/v, zugesetzt wird,
und/oder wobei in dem ersten Schritt die erste Komponente (A) in einer Konzentration im Bereich von 10 bis 35 % w/v, vorzugsweise im Bereich von 20 bis 30 % w/v, in jedem Fall pro Volumen des organischen Lösungsmittels gelöst wird.

13. Verwendung einer Vorrichtung (7) gemäß einem der vorstehenden Ansprüche 1 bis 10 zur Herstellung einer implantierbaren Vorrichtung,
wobei in einem ersten Schritt eine implantierbare Vorrichtung (3), vorzugsweise eine nicht abbaubare implantierbare Vorrichtung (3), zumindest teilweise von mindestens einer solchen Vorrichtung (7) bedeckt oder umgeben wird,
und wobei vorzugsweise in einem zweiten Schritt diese durch eine Öffnung (2) in einen nassen oder feuchten Beutel (1) aus mindestens einem selbsttragenden Hydrogel, Cellulose oder Kollagen (1) eingeführt wird.

14. Kit of parts zur Verwendung gemäß dem vorstehenden Anspruch, umfassend
mindestens einen feuchten oder nassen Beutel (1), vorzugsweise in Form eines Beutels aus mindestens einem selbsttragenden Hydrogel, Cellulose oder Kollagen (1), mit einer Öffnung (2) in einer ersten feuchten Verpackung;
und mindestens eine Vorrichtung (7) gemäß einem der vorstehenden Ansprüche 1 bis 10 in einer separaten feuchten oder trockenen Verpackung, vorzugsweise in einer trockenen Verpackung.

15. Kit of parts gemäß Anspruch 14, wobei mindestens zwei Vorrichtungen (7) in der separaten Verpackung vorgesehen sind und wobei die Vorrichtungen vorzugsweise die Form von rechteckigen oder quadratischen Platten mit einer Länge und/oder Breite im Bereich von 50 bis 110 mm haben, vorzugsweise im Bereich von 60 bis 90 mm, wobei sie vorzugsweise die Form eines Rechtecks mit einer Länge im Bereich von 70 bis 110 mm, vorzugsweise im Bereich von 80 bis 90 mm, und einer Breite im Bereich von 50 bis 90 mm, vorzugsweise im Bereich von 60 bis 80 mm, haben
und/oder wobei die erste Verpackung vorzugsweise Elemente zur Aufrechterhaltung einer kontrollierten Feuchtigkeit umfasst, vorzugsweise in Form von Beuteln aus Aluminium oder Aluminium-Kunststoff-Laminat,
und/oder wobei die separate Verpackung Elemente zum Aufrechterhalten der Trockenheit umfasst, vorzugsweise Trockenmittelelemente.

## Revendications

1. Dispositif (7) pour la libération contrôlée prolongée d'au moins deux substances thérapeutiquement actives après implantation dudit dispositif seul ou en combinaison avec d'autres éléments implantables (1, 3), ledit dispositif (7) comprenant au moins une couche (8) de matériau comprenant au moins deux substances thérapeutiquement actives et assurant la libération contrôlée prolongée desdites au moins deux substances dans des conditions physiologiques,
dans lequel ladite couche (8) comprend une matrice biodégradable constituée d'un mélange de
| | |
|---|---|
| 75 à 95 % | en poids sec d'un premier composant (A) sous la forme d'un copolymère de DL-lactide et de glycolide (PLGA) avec un rapport molaire du DL-lactide au glycolide compris entre 45:55 et 55:45 et avec un poids moléculaire moyen en poids (MW_{w}) compris entre 100 000 et 150 000 g/mol et |
| 5 à 25 % | en poids sec d'un deuxième composant (B) sous forme de polyéthylène glycol (PEG) avec un poids moléculaire moyen en poids (MW_{w}) compris entre 3 000 et 5 500 g/mol, |
les poids secs des composants (A) et (B) se complétant pour atteindre 100 % de ladite matrice,
dans laquelle au moins deux substances thérapeutiquement actives sont dissoutes et/ou en suspension
dans laquelle, parmi les deux substances thérapeutiquement actives, l'une est hydrophobe avec une solubilité inférieure à 5 mg/ml et l'autre est hydrophile avec une solubilité supérieure à 20 mg/ml, dans chaque cas mesurée à un pH de 7 et à une température ambiante de 20 °C,
dans laquelle les au moins deux substances thérapeutiquement actives sont choisies parmi au moins deux antibiotiques choisis dans le groupe des tétracyclines, des pénicillines, des macrolides, des ansamycines, et dans laquelle le total des substances thérapeutiquement actives dans la couche (8) est compris dans la plage de 25 à 40 % en poids/poids sec par rapport au total de la couche (8).

2. Dispositif (7) selon la revendication 1, dans lequel ledit premier composant (A) a un rapport molaire du DL-lactide au glycolide compris entre 48:52 et 52:48, ou d'environ 50:50.

3. Dispositif (7) selon l'une quelconque des revendications précédentes, dans lequel ledit premier composant (A) a un poids moléculaire moyen en poids (MW_{w}) compris entre 110 000 et 140 000 g/mol, de préférence compris entre 120 000 et 130 000 g/mol,
et/ou dans lequel ledit premier composant (A) a un poids moléculaire moyen en nombre (MWₙ) compris entre 50 000 et 100 000 g/mol, de préférence compris entre 60 000 et 80 000 g/mol, de préférence encore compris entre 65 000 et 75 000 g/mol,
et/ou dans lequel ledit premier composant (A) a un point médian de viscosité inhérente compris entre 0,8 et 1,2 dl/g, de préférence compris entre 0,9 et 1,1 dl/g (dans chaque cas mesuré dans du chloroforme, à 25 °C, c = 0,1 g/dl).

4. Dispositif (7) selon l'une quelconque des revendications précédentes, dans lequel ledit deuxième composant (B) a un poids moléculaire moyen en poids (MW_{w} ) compris entre 3 200 et 4 800 g/mol, de préférence entre 3 700 et 4 400 g/mol,
et/ou dans lequel ledit deuxième composant (B) a un point de solidification compris entre 45 et 68 °C, de préférence entre 50 et 65 °C, et de manière particulièrement préférée entre 55 et 60 °C.

5. Dispositif (7) selon l'une quelconque des revendications précédentes, dans lequel ladite couche (8) comprend une matrice constituée d'un mélange de
75 à 92 % en poids sec, de préférence 80 à 90 % en poids sec, de préférence encore 84 à 88 % en poids sec du composant (A) et
8 à 25 % en poids sec, de préférence 10 à 20 % en poids sec, de préférence encore 12 à 16 % en poids sec du composant (B)
les poids secs des composants (A) et (B) se complétant pour atteindre 100 % de ladite matrice.

6. Dispositif (7) selon l'une quelconque des revendications précédentes, dans lequel les au moins deux substances thérapeutiquement actives sont choisies dans le groupe constitué par la tétracycline, la chlortétracycline, l'oxytétracycline, la déméclocycline, la lymécycline, la méclocycline, la méthacycline, la minocycline, en particulier sous forme de chlorhydrate de minocycline, rolitétracycline, doxycycline, tigécycline, éravacycline, sarecycline, omadacycline, rifampicine.

7. Dispositif (7) selon la revendication 6, dans lequel les au moins deux substances thérapeutiquement actives sont une combinaison de minocycline, en particulier sous forme de chlorhydrate de minocycline, et de rifampicine.

8. Dispositif (7) selon l'une quelconque des revendications précédentes, dans lequel la couche (8) en tant qu'au moins deux substances thérapeutiquement actives comprend une combinaison de minocycline, en particulier sous forme de chlorhydrate de minocycline, et de rifampicine dans une concentration totale de 25 à 40 % en poids, de préférence dans la plage de 25 à 35 % en poids, dans chaque cas exprimée en % p/p sec par rapport au total de la couche,
dans laquelle, de préférence, la concentration en minocycline, en particulier sous forme de chlorhydrate de minocycline, est comprise entre 10 et 20 % en poids, de préférence entre 14 et 18 %,
et/ou dans lequel la concentration en rifampicine est comprise entre 15 et 25 % en poids, de préférence entre 17 et 22 % en poids, dans chaque cas exprimée en % p/p sec par rapport au total de la couche.

9. Dispositif (7) selon l'une quelconque des revendications précédentes, dans lequel ladite couche (8) comprend une matrice constituée d'un mélange de
84 à 88 % en poids sec d'un premier composant (A) ayant un poids moléculaire moyen en poids compris entre 120 000 et 130 000 g/mol,
et
12 à 16 % en poids sec d'un deuxième composant (B) ayant un poids moléculaire moyen en poids MWw compris entre 3 700 et 4 400 g/mol,
les poids secs des composants (A) et (B) se complétant pour atteindre 100 % de ladite couche (8),
et dans laquelle la couche (8) comprend au moins deux substances thérapeutiquement actives comprenant une combinaison de minocycline, en particulier sous la forme de chlorhydrate de minocycline, et de rifampicine dans une concentration totale de 25 à 35 % en poids,
la concentration en minocycline, en particulier sous forme de chlorhydrate de minocycline, étant comprise entre 14 et 18 %, et la concentration en rifampicine étant comprise entre 17 et 22 % en poids,
dans chaque cas exprimée en % p/p sec par rapport au total de la couche.

10. Dispositif (7) selon l'une quelconque des revendications précédentes, dans lequel il se présente sous la forme d'au moins un ou d'un seul film autoportant (8), d'un treillis, d'une structure non tissée ou d'une combinaison de ceux-ci, ayant de préférence une épaisseur comprise entre 2,5 et 100 µm, de préférence comprise entre 10 et 30 µm,
dans lequel il prend en outre de préférence la forme d'un film ou d'un patch ayant une extension maximale dans une direction latérale de 30 cm, de préférence une extension comprise entre 0,5 et 15 cm, de préférence comprise entre 1 et 10 cm,
dans lequel, de préférence, le film ou le patch prend une forme ronde, ovale ou polygonale, de préférence une forme rectangulaire ou carrée, éventuellement avec des bords arrondis, et
et/ou dans lequel le dispositif peut être constitué d'une seule ou de plusieurs desdites couches (8), en combinaison avec au moins une couche de support supplémentaire (9), ou de préférence, le dispositif (7) est constitué d'une seule couche (8).

11. Procédé de fabrication d'un dispositif (7) selon l'une quelconque des revendications précédentes, dans lequel
Dans une première étape, ledit premier composant (A) est entièrement dissous dans un solvant organique, de préférence choisi parmi le groupe comprenant l'acétonitrile, le toluène, l'anisole, le chloroforme, le dichlorométhane, le diméthylformamide, le diméthylsulfoxyde, l'acétate d'éthyle, le dioxane, le tétrahydrofurane, le toluène ou une combinaison de ceux-ci,
de préférence sous agitation à une température comprise entre 15 et 30 °C, de préférence à température ambiante (20-25 °C),
pour former une solution dudit premier composant (A),
et dans lequel
dans une deuxième étape, ledit deuxième composant (B) et les au moins deux substances thérapeutiquement actives sont ajoutés à ladite solution dudit premier composant (A),
de préférence sous agitation à une température comprise entre 15 et 30 °C, de préférence à température ambiante,
suivie de l'évaporation d'au moins un solvant pour former le dispositif, de préférence dans un procédé de coulée en solution ou d'enduction en solution, de préférence sur un matériau antiadhésif.

12. Procédé selon la revendication précédente, dans lequel, après ladite première étape et avant de réaliser ladite deuxième étape, un autre solvant organique, différent du solvant organique utilisé dans la première étape, est ajouté à la solution dudit premier composant (A), dans lequel, de préférence, ledit autre solvant organique est choisi dans le groupe constitué par l'acétonitrile, le chlorobenzène, le chloroforme, le cumène, le cyclohexane, 1,2-dichloroéthène, dichlorométhane, 1,2-diméthoxyéthane, N,N-diméthylacétamide, N,N-diméthylformamide, 1,4-dioxane, 2-éthoxyéthanol, éthylèneglycol, formamide, hexane, méthanol, 2-méthoxyéthanol, méthylbutylcétone, méthylcyclohexane, méthylisobutylcétone, N-méthylpyrrolidone, nitrométhane, pyridine, sulfolane, tétrahydrofurane, tétraline, toluène, 1,1,2-trichloroéthène, xylène, acide acétique, heptane, acétone, acétate d'isobutyle, anisole, acétate d'isopropyle, 1-butanol, acétate de méthyle, 2-butanol, 3-méthyl-1-butanol, acétate de butyle, méthyléthylcétone, tert-butylméthyléther, 2-méthyl-1-propanol, diméthylsulfoxyde, pentane, éthanol, 1-pentanol, acétate d'éthyle, 1-propanol, éther éthylique, 2-propanol, formiate d'éthyle, acétate de propyle, acide formique, triéthylamine, 1,1-diéthoxypropane, 1,1-diméthoxyméthane, 2,2-diméthoxypropane, isooctane, éther isopropylique, méthylisopropylcétone, méthyltétrahydrofurane, éther de pétrole, acide trichloracétique, acide trifluoroacétique, ou une combinaison de ceux-ci,
dans lequel, de préférence, ledit autre solvant est ajouté à la solution dudit premier composant (A) dans une proportion comprise entre 20 et 40 % v/v, de préférence entre 25 et 35 % v/v,
et/ou dans lequel, dans la première étape, ledit premier composant (A) est dissous à une concentration comprise entre 10 et 35 % p/v, de préférence entre 20 et 30 % p/v, dans chaque cas d' , par volume de solvant organique.

13. Utilisation d'un dispositif (7) selon l'une quelconque des revendications 1 à 10 précédentes pour la préparation d'un dispositif implantable,
dans lequel, dans une première étape, un dispositif implantable (3), de préférence un dispositif implantable non dégradable (3), est au moins partiellement recouvert ou entouré par au moins un tel dispositif (7),
et dans lequel, de préférence, dans une deuxième étape, celui-ci est inséré dans une poche humide ou mouillée (1) d'au moins un hydrogel autoportant, de cellulose ou de collagène (1) à travers une ouverture (2) de celle-ci.

14. Kit of parts destiné à être utilisé selon la revendication précédente, comprenant
au moins une poche humide ou mouillée (1), de préférence sous la forme d'une poche d'au moins un des éléments suivants : hydrogel autoportant, cellulose ou collagène (1), comportant une ouverture (2) dans un premier emballage humide ;
et au moins un dispositif selon l'une quelconque des revendications 1 à 10 précédentes (7) dans un emballage humide ou sec séparé, de préférence dans un emballage sec.

15. Kit of parts selon la revendication 14, dans lequel il est prévu au moins deux dispositifs (7) dans ledit emballage séparé, et dans lequel les dispositifs prennent de préférence la forme de feuilles rectangulaires ou quadratiques dont la longueur et/ou la largeur est comprise entre 50 et 110 mm, de préférence comprise entre 60 et 90 mm, dans lequel ils se présentent de préférence sous la forme d'un rectangle ayant une longueur comprise entre 70 et 110 mm, de préférence comprise entre 80 et 90 mm, et une largeur comprise entre 50 et 90 mm, de préférence comprise entre 60 et 80 mm
et/ou dans lequel, de préférence, ledit premier emballage comprend des éléments permettant de maintenir une humidité contrôlée, de préférence sous la forme de sachets en aluminium ou en laminé aluminium-plastique,
et/ou dans lequel ledit emballage séparé comprend des éléments permettant de maintenir la sécheresse, de préférence des éléments dessiccants.
